(19) Europäisches Patentamt / European Patent Office / Office européen des brevets

(11) **EP 4 647 521 B1**

(12) **EUROPEAN PATENT SPECIFICATION**

(45) Date of publication and mention of the grant of the patent:
**10.06.2026 Bulletin 2026/24**

(21) Application number: **25169395.8**

(22) Date of filing: **09.04.2025**

(51) International Patent Classification (IPC):
*C22B 26/20* $^{(2006.01)}$    *C01D 15/08* $^{(2006.01)}$
*C22B 7/00* $^{(2006.01)}$    *H01M 10/54* $^{(2006.01)}$
*H01M 10/052* $^{(2010.01)}$    *C22B 26/12* $^{(2006.01)}$
*C22B 3/08* $^{(2006.01)}$    *C22B 3/10* $^{(2006.01)}$
*C01D 15/02* $^{(2006.01)}$    *C22B 3/44* $^{(2006.01)}$

(52) Cooperative Patent Classification (CPC):
**H01M 10/54; C01D 15/02; C01D 15/08; C22B 3/08;
C22B 3/10; C22B 3/44; C22B 7/006; C22B 26/12;
C22B 26/20; H01M 10/052;** Y02W 30/84

(54) **METHOD OF REMOVING CALCIUM IN A RECHARGEABLE LITHIUM BATTERY RECYCLING PROCESS**

VERFAHREN ZUR ENTFERNUNG VON CALCIUM IN EINEM WIEDERAUFBEREITUNGSVERFAHREN FÜR WIEDERAUFLADBARE LITHIUMBATTERIEN

PROCÉDÉ D'ÉLIMINATION DU CALCIUM DANS UN PROCESSUS DE RECYCLAGE DE BATTERIE AU LITHIUM RECHARGEABLE

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: **07.05.2024 KR 20240060066**

(43) Date of publication of application:
**12.11.2025 Bulletin 2025/46**

(73) Proprietor: **SAMSUNG SDI CO., LTD.**
**Yongin-si, Gyeonggi-do 17084 (KR)**

(72) Inventors:
• **YOO, Kwangyong**
  **17084 Yongin-si (KR)**
• **YOU, Yongchan**
  **17084 Yongin-si (KR)**
• **LEE, Youngjun**
  **17084 Yongin-si (KR)**
• **PARK, Seongin**
  **17084 Yongin-si (KR)**
• **SOHN, Myungbeom**
  **17084 Yongin-si (KR)**

(74) Representative: **Marks & Clerk LLP**
**15 Fetter Lane**
**London EC4A 1BW (GB)**

(56) References cited:
CN-A- 101 205 298    CN-A- 107 739 040
CN-A- 116 216 674

**Description**

**BACKGROUND**

**1. Field**

This disclosure relates to a method of removing calcium in a rechargeable lithium battery recycling process.

**2. Description of the Related Art**

Rechargeable lithium batteries may be recharged and have three or more times energy density per unit weight as compared to a conventional lead storage battery, nickel-cadmium battery, nickel hydrogen battery, nickel zinc battery and the like. Rechargeable lithium batteries may be also charged at a high rate and, thus, are commercially manufactured for laptop computers, cell phones, electric tools, electric bikes, and the like. There is also active research providing additional energy density in rechargeable lithium batteries.

Rechargeable lithium batteries are made by injecting an electrolyte solution into an electrode assembly that includes a positive electrode including a positive electrode active material and a negative electrode including a negative electrode active material.

Because lithium is a key raw material for the rechargeable lithium batteries, it has been continuously increasing in value in recent times. Many companies are conducting research on recycling lithium in view of its increasing cost.

However, because liquid obtained from a recycling process of rechargeable lithium batteries contains a large amount of impurities, it is difficult to extract lithium itself. In particular, an important part of the lithium recycling process is removing calcium, which is re-dissolved in an alkali solution, from the liquid obtained in the recycling process of rechargeable lithium batteries. CN107739040A discloses a method for recovering lithium carbonate from a lithium-containing waste solution and is representative of the state of the art.

**SUMMARY**

Embodiments of the present disclosure effectively remove calcium from liquid obtained in a recycling process of rechargeable lithium batteries and produce a high-purity lithium compound.

Some example embodiments provide a method of removing calcium from a lithium liquid in a rechargeable lithium battery recycling process, the method including recovering acidic lithium liquid including calcium from the recycling process; adding an oxalate aqueous solution to the acidic lithium liquid as a first calcium removal process; raising a pH of the acidic lithium liquid to prepare an alkaline lithium liquid; and adding ammonium oxalate to the alkaline lithium liquid as a second calcium removal process.

At least some of the above and other features of the invention are set out in the claims.

With methods according to embodiments of the present disclosure, calcium can be effectively removed from liquid obtained from rechargeable lithium battery recycling process and a high purity lithium compound can be produced.

**DETAILED DESCRIPTION**

In some example embodiments, a method of removing calcium includes recovering acidic lithium liquid that includes calcium from a recycling process of a rechargeable lithium battery; adding (e.g., injecting) an oxalate aqueous solution (e.g., an aqueous solution of oxalic acid) into the acidic lithium liquid as a first calcium removal; raising pH of the acidic lithium liquid and preparing an alkaline lithium liquid; and adding ammonium oxalate to the alkaline lithium liquid as a second calcium removal.

In the method of removing calcium according to some example embodiments, calcium is removed twice for each pH section of the liquid obtained from the recycling process of rechargeable lithium batteries. Thus, calcium can be effectively removed and a high-purity lithium compound can be produced.

Hereinafter, a method for removing calcium according to example embodiments will be described in detail, step by step.

Recovery process of acidic lithium liquid including calcium

Acidic lithium liquid including calcium can be recovered from a recycling process of rechargeable lithium batteries.

The acidic lithium liquid including calcium may have a pH of about 1 to about 5, or, in more particular embodiments, a pH of about 2 to about 4.

The acidic lithium liquid including calcium may also include lithium sulfate, lithium hydrochloride (e.g., lithium chloride), or a combination thereof. The calcium in the acidic lithium liquid including calcium may be present in an ionic state, and the calcium ion may be in an amount of about 1 ppm to about 10,000 ppm, about 1 ppm to about 1,000 ppm, or about 10 ppm to about 500 ppm.

First calcium removal process

After recovering the acidic lithium liquid including calcium, an aqueous oxalate solution (e.g., an aqueous solution of oxalic acid) may be added to the acidic lithium liquid as a first calcium removal process.

The step of first calcium removal may be performed at about 0 °C to about 80 °C, about 30 °C to about 70 °C, or about 40 °C to about 60 °C. The oxalate aqueous solution may be added at a temperature of about 0 °C to about 80 °C, about 30 °C to about 70 °C, or about 40 °C to about 60 °C.

A concentration of the oxalate aqueous solution (e.g., an aqueous solution of oxalic acid) may be about 0.1 M to about 1 M. Such a concentration takes into account the content of calcium ions in the acidic lithium liquid including calcium.

In the step of removing calcium, calcium ions and oxalate ions in the acidic lithium liquid react and precipitate in the form of calcium oxalate. The calcium oxalate may be removed by using filtration methods widely known in the art.

pH adjustment process

By increasing the pH of the acidic lithium liquid after the first calcium removal process, an alkaline lithium liquid may be produced. To prepare the alkaline lithium liquid, sodium hydroxide, sodium carbonate, or a combination thereof may be used.

The alkaline lithium liquid may have a pH of about 7 to about 14, or a pH of about 8 to about 12.

Other impurities removal process

After the step of preparing the acidic or alkaline lithium liquid (e.g., after the step of preparing the acidic lithium liquid), a step of removing iron, magnesium, zinc, or a combination thereof may be performed.

For example, about 1 to about 5 parts by weight of sodium carbonate ($Na_2CO_3$) is added to 100 parts by weight of the acidic or alkaline lithium liquid, and then a basic material such as sodium hydroxide (NaOH), potassium hydroxide (KOH), and lithium hydroxide (LiOH) may be added. Accordingly, high purity lithium compounds can be recovered in the final process.

Second calcium removal process

A step of second removing of calcium may be performed at about 40 °C to about 100 °C, about 50 °C to about 90 °C, or about 60 °C to about 80 °C. For this process, ammonium oxalate aqueous solution can be added at a temperature of about 20 °C to about 90 °C, about 40 °C to about 85 °C, or about 60 °C to about 80 °C.

In the second removal of calcium step, calcium ions and oxalate ions in the basic lithium liquid react and calcium precipitates in the form of calcium oxalate. The calcium oxalate may be removed using filtration methods widely known in the art.

Post-process after calcium removal

After the second calcium removal step, high purity lithium compounds can be obtained in various forms.

For example, the method may further include adding a carbonate ion-containing material or carbon dioxide gas to the alkaline lithium liquid from which the calcium is removed to produce lithium carbonate. The carbonate ion-containing material may be, for example, sodium carbonate ($Na_2CO_3$).

In another example, the method may further include adding a hydroxide ion-containing material to the alkaline lithium liquid from which the calcium is removed and concentrating and crystallizing to produce lithium hydroxide. The carbonate ion-containing material may be, for example, sodium hydroxide (NaOH), potassium hydroxide (KOH), lithium hydroxide (LiOH), etc.

The high-purity lithium compound obtained as described above can be used as a lithium source for a positive electrode active material. Methods of preparing the positive electrode active material are generally known in the art.

In some embodiments, the method of removing calcium from a lithium liquid from a rechargeable lithium battery recycling process may consist of:

recovering acidic lithium liquid including calcium from the recycling process;

adding an oxalate aqueous solution to the acidic lithium liquid as a first calcium removal process;
removing at least one of iron, magnesium, and zinc;
raising a pH of the acidic lithium liquid to prepare an alkaline lithium liquid;
adding ammonium oxalate to the alkaline lithium liquid as a second calcium removal process; and
adding a carbonate ion-containing material or carbon dioxide gas to the alkaline lithium liquid to produce lithium carbonate. Aspects of each step are described in the disclosure above.

In some embodiments, the method of removing calcium from a lithium liquid from a rechargeable lithium battery recycling process may consist of:

recovering acidic lithium liquid including calcium from the recycling process;
adding an oxalate aqueous solution to the acidic lithium liquid as a first calcium removal process;
removing at least one of iron, magnesium, and zinc;
raising a pH of the acidic lithium liquid to prepare an alkaline lithium liquid;
adding ammonium oxalate to the alkaline lithium liquid as a second calcium removal process; and
adding a hydroxide ion-containing material to the alkaline lithium liquid, concentrating, and crystallizing to produce lithium hydroxide. Aspects of each step are described in the disclosure above.

Examples and comparative examples are described below. However, the following are only examples of the present disclosure, and the present disclosure is not limited to the following examples.

**Example 1**

(1) Recovery process of acidic lithium liquid including calcium

Acidic lithium liquid including calcium was recovered from a recycling process of a rechargeable lithium battery. The acidic lithium liquid included 500 ppm of calcium ions and 15,000 mg/L of lithium sulfate, and the liquid had pH 2.

(2) First calcium removal process

An aqueous solution of oxalic acid was prepared at a concentration of 0.5 M at 60 °C and then added to the acidic lithium liquid while maintaining temperature.
Calcium ions in the acidic lithium liquid reacted with oxalate ions to precipitate calcium oxalate. Then, the calcium oxalate was filtered to separate solid and liquid, and, after removing the solid, the liquid was obtained. Herein, the obtained liquid will be referred to as "acidic lithium liquid from which calcium was once removed."

(3) Other impurities removal process

After the preparing of the acidic lithium liquid from which calcium was removed, 1 part by weight of sodium carbonate ($Na_2CO_3$, purity: 99%) based on 100 parts by weight of the acidic lithium liquid was added to remove iron, magnesium, zinc, etc.

(4) pH adjustment process

After removing other impurities from the acidic lithium liquid, sodium hydroxide was added to increase the pH to 11 and, thus, obtain "alkaline lithium liquid from which calcium was once removed."

(5) Second calcium removal process

An ammonium oxalate aqueous solution was prepared at a concentration of 0.5 M at 80 °C and then added to the alkaline lithium liquid while maintaining temperature.
Calcium ions in the alkaline lithium liquid reacted with oxalate ions to precipitate calcium oxalate. Then the calcium oxalate was filtered to separate solid and liquid, and after removing the solid, the liquid was obtained. Herein, the obtained liquid will be referred to as "alkaline lithium liquid from which calcium was twice removed."

(6) Post-process after calcium removal

Sodium carbonate ($Na_2CO_3$) was added to the alkaline lithium liquid from which the calcium was twice removed to obtain lithium carbonate.

## Example 2

Lithium carbonate was prepared by twice removing calcium from the acidic lithium liquid including calcium in the same manner as in Example 1, except that the temperature of the first calcium removal process was changed to 40 °C.

## Example 3

Lithium carbonate was prepared by twice removing calcium from the acidic lithium liquid including calcium in the same manner as in Example 1, except that the temperature of the first calcium removal was changed to 50 °C.

## Example 4

Lithium carbonate was prepared by twice removing calcium from the acidic lithium liquid including calcium in the same manner as in Example 1, except that the temperature of the second calcium removal process was changed to 70 °C.

## Example 5

Lithium carbonate was prepared by twice removing calcium from the acidic lithium liquid including calcium in the same manner as in Example 1, except that the temperature of the second calcium removal process was changed to 60 °C.

## Comparative Example 1 (Ref.)

Lithium carbonate was prepared from acidic lithium liquid including calcium by adding sodium carbonate ($Na_2CO_3$) in a recycling process of rechargeable lithium batteries without a calcium-removing process and without a pH-adjusting process.

## Comparative Example 2

Lithium carbonate was prepared from acidic lithium liquid including calcium by adding sodium carbonate ($Na_2CO_3$) after performing the first calcium removal process alone in a recycling process of rechargeable lithium batteries in the same manner as in Example 1.

## Comparative Example 3

Lithium carbonate was prepared from the acidic lithium liquid including calcium by adding sodium carbonate ($Na_2CO_3$) after performing the pH-adjusting process and the second calcium removal process in the same manner as in Example 1 without the first calcium removal process in a recycling process of rechargeable lithium batteries.

## Comparative Example 4

Lithium carbonate was prepared from the acidic lithium liquid including calcium by adding sodium carbonate ($Na_2CO_3$) after performing the first calcium removal process and the second calcium removal process in the same manner as in Example 1 without the pH-adjusting process in a recycling process of rechargeable lithium batteries.

For reference, the processes performed in Examples 1 to 5 and Comparative Examples 1 to 4 are summarized in Table 1.

(Table 1)

| | Addition of oxalic acid (first calcium removal) | | pH adjustment | | | Addition of ammonium oxalic acid (second calcium removal) | |
|---|---|---|---|---|---|---|---|
| | Added or not | Temperature | Added or not | Before adjustment | After adjustment | Added or not | Temperature |
| Ex. 1 | ○ | 60 °C | ○ | pH 2 | pH 11 | ○ | 80 °C |

(continued)

| | Addition of oxalic acid (first calcium removal) | | pH adjustment | | | Addition of ammonium oxalic acid (second calcium removal) | |
|---|---|---|---|---|---|---|---|
| | Added or not | Tempe rature | Added or not | Before adjust ment | After adjust ment | Added or not | Tempe rature |
| Ex. 2 | ○ | 40 °C | ○ | pH 2 | pH 11 | ○ | 80 °C |
| Ex. 3 | ○ | 50 °C | ○ | pH 2 | pH 11 | ○ | 80 °C |
| Ex. 4 | ○ | 60 °C | ○ | pH 2 | pH 11 | ○ | 70 °C |
| Ex. 5 | ○ | 60 °C | ○ | pH 2 | pH 11 | ○ | 60 °C |
| Comp. Ex. 1 (Ref.) | X | - | X | pH 2 | pH 2 | X | - |
| Comp. Ex. 2 | ○ | 60 °C | X | pH 2 | pH 2 | X | - |
| Comp. Ex. 3 | X | - | ○ | pH 2 | pH 11 | ○ | 80 °C |
| Comp. Ex. 4 | ○ | 60 °C | X | pH 2 | pH 2 | ○ | 80 °C |

**Evaluation Example 1: Evaluation of calcium removal rate**

In Comparative Example 1, the acidic lithium liquid including calcium from the recycling process of rechargeable lithium batteries was measured with respect to a content of calcium ions by using inductively coupled plasma (ICP). This measurement value will be referred to as "first calcium ion content."

Each liquid from which calcium was removed according to Examples 1 to 5 and Comparative Examples 2 to 4 was measured with respect to a content of calcium ions in the same manner. This measurement value will be referred to as "second calcium ion content."

The first calcium ion content and the second calcium ion content were inserted into Equation 1 to evaluate a calcium removal rate, and the results are shown in Table 2.

Calcium removal rate (%) = [(first calcium ion content)-(second calcium ion content)]/(first calcium ion content) [Equation 1]

**Evaluation Example 2: Purity evaluation of lithium carbonate**

The lithium carbonates obtained in Examples 1 to 5 and Comparative Examples 1 to 4 were evaluated with respect to purity by using lithium titration techniques. The evaluation results are shown in Table 2.

(Table 2)

| | Calcium removal rate | Lithium carbonate purity |
|---|---|---|
| Example 1 | 99% | 99.99% |
| Example 2 | 99% | 99.99% |
| Example 3 | 99% | 99.99% |
| Example 4 | 99% | 99.99% |
| Example 5 | 99% | 99.99% |
| Comparative Example 1 (Ref.) | 0% | 98% |
| Comparative Example 2 | 99% | 99.00% |
| Comparative Example 3 | 0% | 99.00% |
| Comparative Example 4 | 99% | 99.90% |

**Summary**

Referring to Tables 1 and 2, as compared with the case of not removing calcium (Comparative Example 1) or the case of once removing calcium (Comparative Examples 2 and 3), Examples 1 to 5 effectively removed calcium and resulted in lithium compounds with high purity.

On the other hand, Comparative Example 4, in which calcium was twice removed only in the acidic state without the pH-adjusting process exhibited improved effects, but lower effects than those of Examples 1 to 5.

Accordingly, methods of removing calcium according to example embodiments, such as Examples 1 to 5, in which calcium is twice removed in each pH section of liquid obtained from a recycling process of rechargeable lithium batteries, effectively removes calcium and provides a lithium compound with high purity.

While this disclosure has been described in connection with what is presently considered to be practical example embodiments, it is to be understood that the invention is not limited to the disclosed embodiments.

**Claims**

1. A method of removing calcium from a lithium liquid from a rechargeable lithium battery recycling process, the method comprising:

   recovering acidic lithium liquid including calcium from the recycling process;
   adding an oxalate aqueous solution to the acidic lithium liquid as a first calcium removal process;
   raising a pH of the acidic lithium liquid to prepare an alkaline lithium liquid; and
   adding ammonium oxalate to the alkaline lithium liquid as a second calcium removal process.

2. The method as claimed in claim 1, wherein the acidic lithium liquid has pH of about 1 to about 5 before the pH is raised.

3. The method as claimed in claim 1 or claim 2, wherein the acidic lithium liquid includes at least one of lithium sulfate and lithium hydrochloride.

4. The method as claimed in any one of claims 1 to 3, wherein, before the first calcium removal process, the calcium in the acidic lithium liquid is present in an ionic state and the calcium ion is included in an amount of about 1 to about 10,000 ppm.

5. The method as claimed in any one of claims 1 to 4, wherein the oxalate aqueous solution has a concentration of about 0.1 M to about 1 M.

6. The method as claimed in any one of claims 1 to 5, wherein the first calcium removal process is performed at a temperature range of about 0 °C to about 80 °C.

7. The method as claimed in any one of claims 1 to 6, wherein calcium is precipitated as calcium oxalate in the first calcium removal process.

8. The method as claimed in any one of claims 1 to 7, wherein at least one of sodium hydroxide and sodium carbonate is used to prepare the alkaline lithium liquid.

9. The method as claimed in any one of claims 1 to 8, wherein, after the preparing of the acidic lithium liquid, at least one of iron, magnesium, and zinc is removed.

10. The method as claimed in any one of claims 1 to 9, wherein the alkaline lithium liquid has a pH of about 7 to about 14.

11. The method as claimed in any one of claims 1 to 10, wherein the second calcium removal process is performed at a temperature range of about 40 °C to about 100 °C.

12. The method as claimed in any one of claims 1 to 11, wherein calcium is precipitated in a form of calcium oxalate in the second calcium removal process.

13. The method as claimed in any one of claims 1 to 12, wherein, after the second calcium removal process, the method further includes adding a carbonate ion-containing material or carbon dioxide gas to the alkaline lithium liquid to produce lithium carbonate.

14. The method as claimed in any one of claims 1 to 13, wherein after the second calcium removal process, the method further includes adding a hydroxide ion-containing material to the alkaline lithium liquid, concentrating, and crystallizing to produce lithium hydroxide.


**Patentansprüche**

1. Verfahren zum Entfernen von Calcium aus einer Lithiumflüssigkeit aus einem Recyclingprozess für wiederaufladbare Lithiumbatterien, wobei das Verfahren Folgendes umfasst:

   Zurückgewinnen von saurer Lithiumflüssigkeit, die Calcium einschließt, aus dem Recyclingprozess;
   Zugeben einer wässrigen Oxalatlösung zur sauren Lithiumflüssigkeit als ein erster Calciumentfernungsprozess;
   Erhöhen eines pH-Werts der sauren Lithiumflüssigkeit, um eine alkalische Lithiumflüssigkeit herzustellen; und
   Zugeben von Ammoniumoxalat zur alkalischen Lithiumflüssigkeit als ein zweiter Calciumentfernungsprozess.

2. Verfahren nach Anspruch 1, wobei die saure Lithiumflüssigkeit, bevor der pH-Wert erhöht wird, einen pH-Wert von etwa 1 bis etwa 5 aufweist.

3. Verfahren nach Anspruch 1 oder Anspruch 2, wobei die saure Lithiumflüssigkeit mindestens eines von Lithiumsulfat und Lithiumhydrochlorid einschließt.

4. Verfahren nach einem der Ansprüche 1 bis 3, wobei, vor dem ersten Calciumentfernungsprozess, das Calcium in der sauren Lithiumflüssigkeit in einem ionischen Zustand vorliegt und das Calciumion in einer Menge von etwa 1 bis etwa 10.000 ppm eingeschlossen ist.

5. Verfahren nach einem der Ansprüche 1 bis 4, wobei die wässrige Oxalatlösung eine Konzentration von etwa 0,1 M bis etwa 1 M aufweist.

6. Verfahren nach einem der Ansprüche 1 bis 5, wobei der erste Calciumentfernungsprozess bei einem Temperaturbereich von etwa 0 °C bis etwa 80 °C durchgeführt wird.

7. Verfahren nach einem der Ansprüche 1 bis 6, wobei Calcium im ersten Calciumentfernungsprozess als Calciumoxalat ausgefällt wird.

8. Verfahren nach einem der Ansprüche 1 bis 7, wobei mindestens eines von Natriumhydroxid und Natriumcarbonat verwendet wird, um die alkalische Lithiumflüssigkeit herzustellen.

9. Verfahren nach einem der Ansprüche 1 bis 8, wobei, nach dem Herstellen der sauren Lithiumflüssigkeit, mindestens eines von Eisen, Magnesium und Zink entfernt wird.

10. Verfahren nach einem der Ansprüche 1 bis 9, wobei die alkalische Lithiumflüssigkeit einen pH-Wert von etwa 7 bis etwa 14 aufweist.

11. Verfahren nach einem der Ansprüche 1 bis 10, wobei der zweite Calciumentfernungsprozess bei einem Temperaturbereich von etwa 40 °C bis etwa 100 °C durchgeführt wird.

12. Verfahren nach einem der Ansprüche 1 bis 11, wobei Calcium im zweiten Calciumentfernungsprozess in einer Form von Calciumoxalat ausgefällt wird.

13. Verfahren nach einem der Ansprüche 1 bis 12, wobei, nach dem zweiten Calciumentfernungsprozess, das Verfahren weiter das Zugeben eines carbonationenhaltigen Materials oder von Kohlendioxidgas zu der alkalischen Lithiumflüssigkeit einschließt, um Lithiumcarbonat zu erzeugen.

14. Verfahren nach einem der Ansprüche 1 bis 13, wobei, nach dem zweiten Calciumentfernungsprozess, das Verfahren weiter das Zugeben eines hydroxidionenhaltigen Materials zu der alkalischen Lithiumflüssigkeit, das Konzentrieren und das Kristallisieren einschließt, um Lithiumhydroxid zu erzeugen.

**Revendications**

1. Procédé d'élimination du calcium d'un liquide de lithium issu d'un processus de recyclage d'une batterie au lithium rechargeable, le procédé comprenant :

   la récupération du liquide de lithium acide, y compris du calcium, du processus de recyclage ;
   l'ajout d'une solution aqueuse d'oxalate au liquide de lithium acide comme premier processus d'élimination du calcium ;
   l'augmentation du pH du liquide de lithium acide pour préparer un liquide de lithium alcalin ; et
   l'ajout d'oxalate d'ammonium au liquide de lithium alcalin comme second processus d'élimination du calcium.

2. Procédé selon la revendication 1, dans lequel le liquide de lithium acide présente un pH allant d'environ 1 à environ 5 avant que le pH ne soit augmenté.

3. Procédé selon la revendication 1 ou la revendication 2, dans lequel le liquide de lithium acide inclut au moins un élément parmi le sulfate de lithium et le chlorhydrate de lithium.

4. Procédé selon l'une quelconque des revendications 1 à 3, dans lequel, avant le premier processus d'élimination du calcium, le calcium du liquide de lithium acide est présent à l'état ionique et l'ion calcium est inclus en une quantité allant d'environ 1 à environ 10 000 ppm.

5. Procédé selon l'une quelconque des revendications 1 à 4, dans lequel la solution aqueuse d'oxalate présente une concentration allant d'environ 0,1 M à environ 1 M.

6. Procédé selon l'une quelconque des revendications 1 à 5, dans lequel le premier processus d'élimination du calcium est effectué dans une plage de températures allant d'environ 0 °C à environ 80 °C.

7. Procédé selon l'une quelconque des revendications 1 à 6, dans lequel le calcium est précipité sous forme d'oxalate de calcium lors du premier processus d'élimination du calcium.

8. Procédé selon l'une quelconque des revendications 1 à 7, dans lequel au moins un élément parmi l'hydroxyde de sodium et le carbonate de sodium est utilisé pour préparer le liquide de lithium alcalin.

9. Procédé selon l'une quelconque des revendications 1 à 8, dans lequel, après la préparation du liquide de lithium acide, au moins un élément parmi le fer, le magnésium et le zinc est éliminé.

10. Procédé selon l'une quelconque des revendications 1 à 9, dans lequel le liquide de lithium alcalin présente un pH allant d'environ 7 à environ 14.

11. Procédé selon l'une quelconque des revendications 1 à 10, dans lequel le second processus d'élimination du calcium est effectué dans une plage de températures allant d'environ 40 °C à environ 100 °C.

12. Procédé selon l'une quelconque des revendications 1 à 11, dans lequel le calcium est précipité sous forme d'oxalate de calcium lors du second processus d'élimination du calcium.

13. Procédé selon l'une quelconque des revendications 1 à 12, dans lequel, après le second processus d'élimination du calcium, le procédé inclut en outre l'ajout d'un matériau contenant des ions carbonate ou de dioxyde de carbone gazeux au liquide de lithium alcalin pour produire du carbonate de lithium.

14. Procédé selon l'une quelconque des revendications 1 à 13, dans lequel, après le second processus d'élimination du calcium, le procédé inclut en outre l'ajout d'un matériau contenant des ions hydroxyde au liquide de lithium alcalin, la concentration et la cristallisation pour produire de l'hydroxyde de lithium.

**EP 4 647 521 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 107739040 A